# EUROPÄISCHE PATENTSCHRIFT

(11) **EP 1 466 571 B1**
(45) Veröffentlichungstag und Bekanntmachung des Hinweises auf die Patenterteilung: **23.08.2006**
(21) Anmeldenummer: 04405213.2
(22) Anmeldetag: 07.04.2004
(51) Int. Cl.: A61F 2/16

(54) **Kartusche für eine intraokulare Linse**
Cartridge for an intraocular lens
Cartouche pour lentille intra-oculaire

(30) Priorität: 07.04.2003 US 460416 P
(43) Veröffentlichungstag der Anmeldung: 13.10.2004
(73) Patentinhaber: Anton Meyer & Co.AG, 2560 Nidau (CH); Asico LLC, Westmont, IL 60559 (US)
(72) Erfinder: Meyer, Rolf, 2562 Port (CH)
(74) Vertreter: Clerc, Natalia

(56) Entgegenhaltungen:
- WO-A-01/87186
- WO-A-95/13022
- WO-A-20/04010903
- US-A- 5 304 182
- US-B1- 6 497 708

## Beschreibung

### Technisches Gebiet

Die Erfindung betrifft eine Kartusche für eine intraokulare Linse zur Verwendung in einem Injektor.

### Stand der Technik

In der heutigen Augenchirurgie zählt die Ersetzung einer trüben natürlichen Augenlinse durch eine künstliche Linse zu den Routineeingriffen. Die Einführung selber erfolgt oft mittels eines Injektors, wie er beispielsweise aus US-A-5'643'276 und EP-A-1' 287'791 bekannt ist.

Die Injektoren sind zum Teil mit Mitteln versehen, um die künstliche Linse einfacher in den Injektor einbringen und darin falten zu können. Derartige Mittel sind beispielsweise aus US 6'143'001, US 6'491'697, US 5'944'725, US 6'336'932, US 6'371'960, WO 96/03924 und US 2002/0022881 bekannt.

Ferner sind Kartuschen bekannt, in welche die Linse eingelegt, zusammengefaltet und gemeinsam mit der Kartusche in den Injektor eingeführt werden kann. Derartige Kartuschen offenbart WO 02/058596 und US 5'499'987.

Aus US 5'304'182 ist ein Injektor bekannt, dessen Kopf ein verschiebbares röhrenartiges Element enthält. Dieses Element dient dazu, eine Linse aufzurollen, indem es eine Aufnahmekammer innerhalb des Injektorkopfes begrenzt.

Wichtig bei der Augenoperation ist, dass dem Patientenauge nur ein möglichst kleiner Schnitt zugefügt werden muss, um die Linse einzubringen. Deshalb ist es vorteilhaft, wenn die in das Auge eingeführte Spitze des Injektors möglichst einen kleinen Durchmesser aufweist. Die Grösse des Durchmessers wird jedoch vor allem dadurch bestimmt, wie gross die zusammengefaltete künstliche Linse noch ist, welche bei der Injektion ins Auge durch diese Spitze durchgepresst werden muss.

### Darstellung der Erfindung

Es ist eine Aufgabe der Erfindung, ein Mittel zu schaffen, mit dessen Hilfe eine intraokulare, künstliche Linse auf einfache Weise und ohne die Linse zu beschädigen in ein möglichst kleines Format gebracht werden kann.

Diese Aufgabe löst eine Kartusche mit den Merkmalen des Patentanspruchs 1.

Die erfindungsgemässe Kartusche ermöglicht es, eine ungefaltete deformierbare Linse mit einer einzigen Pinzette auf eine Auflagefläche der Kartusche zu legen und mittels eines in der Kartusche integrierten Schiebeelements die Linse in ein kleineres Format zu bringen. Vorzugsweise wird die Linse beim Schieben gerollt. Vorzugsweise liegt die Linse, wenn das Schiebeelement vollständig eingeschoben ist, bereits in der Durchgangsöffnung, durch welche sie bei der Augenoperation mittels des Injektors ausgestossen wird. Beim Rollen der Linse dient somit die innere Wandung der Durchgangsöffnung als Führung und bestimmt den resultierenden Durchmesser der gerollten Linse.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Gleiche Teile sind mit gleichen Bezugszeichen versehen. Es zeigen:
- Figur 1a: eine perspektivische Ansicht einer nicht zur Erfindung gehörenden Kartusche mit eingelegter Linse, aber noch nicht eingeschobenem Schiebeelement;
- Figur 1b: die Kartusche gemäss Figur 1a mit teilweise eingeschobenem Schiebeelement;
- Figur 1c: die Kartusche gemäss Figur 1a mit gänzlich eingeschobenem Schiebeelement;
- Figur 1d: die Kartusche gemäss Figur 1a bei Gebrauch in einem Injektor;
- Figur 1e: die Kartusche gemäss Figur 1a mit teilweise eingeschobenem Schiebeelement im Teilschnitt dargestellt;
- Figur 1f: einen Längsschnitt durch Figur 1d;
- Figur 2a: eine perspektivische Ansicht einer nicht zur Erfindung gehörenden Kartusche mit eingelegter Linse, aber noch nicht eingeschobenem Schiebeelement;
- Figur 2b: die Kartusche gemäss Figur 2a mit teilweise eingeschobenem Schiebeelement;
- Figur 2c: die Kartusche gemäss Figur 2a mit gänzlich eingeschobenem Schiebeelement;
- Figur 2d: die Kartusche gemäss Figur 2a bei Gebrauch in einem Injektor;
- Figur 2e: die Kartusche gemäss Figur 2a mit teilweise eingeschobenem Schiebeelement im Teilschnitt dargestellt;
- Figur 2f: einen Längsschnitt durch Figur 2d;
- Figur 3a: eine perspektivische Ansicht einer erfindungsgemässen Kartusche mit eingelegter Linse, aber noch nicht eingeschobenem Schiebeelement;
- Figur 3b: die Kartusche gemäss Figur 3a mit teilweise eingeschobenem Schiebeelement;
- Figur 3c: die Kartusche gemäss Figur 3a mit gänzlich eingeschobenem Schiebeelement;
- Figur 3d: die Kartusche gemäss Figur 3a bei Gebrauch in einem Injektor und
- Figur 3e: einen Längsschnitt durch die Kartusche gemäss Figur 3d.

### Wege zur Ausführung der Erfindung

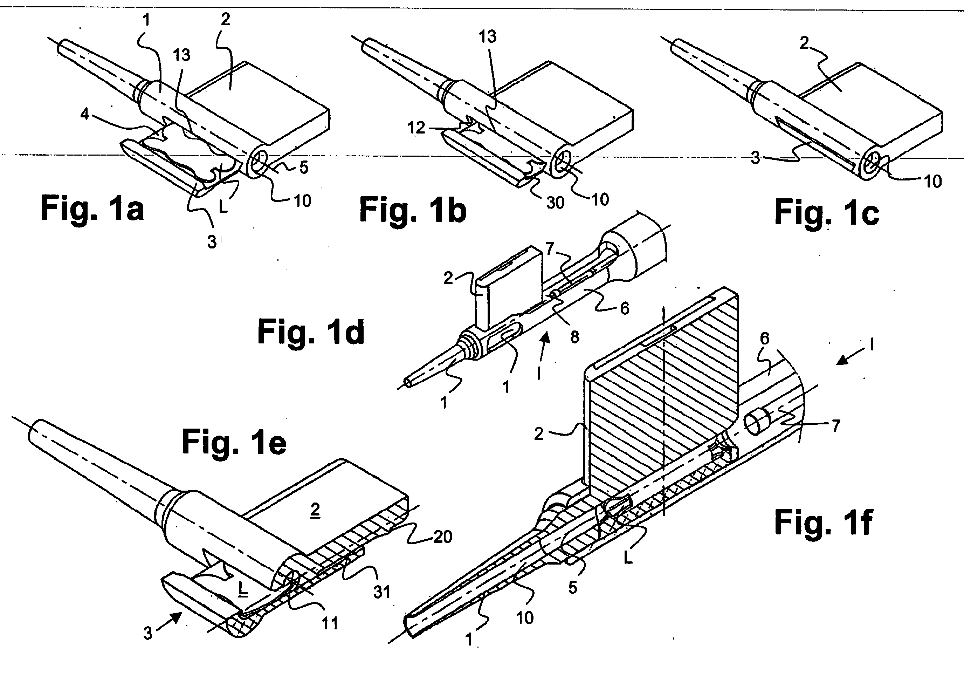

In den Figuren 1a bis 1e ist eine erste, nicht zur Erfindung gehörende Kartusche dargestellt. In diese Kartusche lässt sich eine deformierbare Linse L gefaltet, vorzugsweise zusammengerollt, anordnen. Die Kartusche wird bei Gebrauch in einen Injektor I, genauer in eine Öffnung des Injektorgehäuses 6, eingesetzt und die Linse L wird mittels eines Injektorkolbens 7, welcher innerhalb einer Durchgangsöffnung 8 des Injektors bewegbar ist, durch die Kartusche hindurch in ein Auge eines Patienten injiziert. Dies ist in Figur 1f am besten erkennbar.

Die Kartusche weist einen hohlen, kreiszylinderförmigen Grundkörper 1, eine daran angeordnete Kassette 2, ein Schiebeelement 3, eine Auflagefläche 4 und eine an die Auflagefläche angrenzende gewölbte Fläche 11 auf.

Der Grundkörper 1 definiert eine Längsachse 5 der Kartusche. Entlang dieser Längsachse 5 erstreckt sich eine Durchgangs- oder Durchführungsöffnung 10. Ist die Kartusche im Injektor eingesetzt, fluchtet diese Durchgangsöffnung 10 mit einer Durchgangsöffnung des Injektors. Die gewölbte Fläche 11 wird in diesem Beispiel durch einen Bereich der Durchgangsöffnung 11 gebildet und ist somit begrenzt.

Die Kassette 2 ist entlang einer Längsseite des Grundkörpers 1 an dieser angeordnet. Das Schiebeelement 3 ist in der Kassette 2 verschiebbar geführt angeordnet, wobei es in einer Ebene senkrecht zur Längsachse 5 verschiebbar ist.

Vorzugsweise lässt sich das Schiebeelement 3 ganz entfernen. Es sind jedoch auch Ausführungsformen möglich, bei welchen es verschiebbar in der Kassette 2 gehalten ist, aber nicht zerstörungsfrei aus dieser entfernt werden kann.

Im hier dargestellten Beispiel befindet sich die Auflagefläche 4 auf dem Schiebeelement 3. Die Auflagefläche 4 ist mindestens annähernd plan ausgebildet und dient zur Auflage der Linse L im ungefalteten oder nur teilgefalteten Zustand. Die Auflagefläche 4 ist aus einem Material mit guten Gleiteigenschaften gefertigt oder sie ist mit einem diese Eigenschaft aufweisenden Material beschichtet.

Der Grundkörper 1 weist eine Nut 12 auf, durch welche das Schiebeelement 3 in Richtung Kassette 2 und in diese hinein schiebbar ist. Somit ist eine Schublade vorhanden. Die Nut 12 ist durch eine obere Anschlagkante 13 begrenzt. Wird das Schiebeelement 3 in die Kassette 2 hineingeschoben, gleitet die Linse L unter dieser Anschlagkante 13 hindurch geführt auf dem Schiebeelement entlang. Die Auflagefläche 4 grenzt an die gewölbte Fläche 11 an, so dass die Linse L beim Einschieben des Schiebeelements 3 entsprechend deren Wölbung gefaltet bzw. gerollt wird.

Das Schiebeelement 3 weist vorzugsweise eine Stirn- oder Führungsfläche 30 auf, welche derart ausgebildet ist, dass sie die Linse L schonend verschiebt. Insbesondere kann die Stirnfläche 30 gebogen ausgebildet sein. Sie kann zusätzlich oder alternativ dazu mit einer geeigneten Beschichtung versehen und/oder aus einem geeigneten Material, insbesondere Kunststoff, gefertigt sein. Vorzugsweise ist die restliche Kartusche mindestens teilweise, vorzugsweise ganz, aus Kunststoff gefertigt.

Im gefalteten oder gerollten Zustand der Linse L ist das Schiebeelement 3 vorzugsweise so in die Kassette 2 versenkt, dass sie an der dem Einschubbereich gegenüberliegenden Seite der Kassette 2 der übrigen Wandung der Kartusche nicht vorsteht.

Die Kassette 2 ist als Halteelement beim Einlegen der Kartusche in den Injektor ausgebildet, um das Einlegen und Entfernen der Kartusche zu erleichtern. Die Kartusche lässt sich somit an der Kassette von Hand oder mittels einer Pinzette ergreifen und in den Injektor einlegen bzw. aus diesem wieder entfernen.

In diesem Beispiel ist die Kassette 2 mindestens annähernd geschlossen ausgebildet. In den nachfolgend beschriebenen Beispielen hingegen bildet die Kassette 2 mit dem eingeschobenen Schiebeelement 3 eine mindestens annähernd geschlossene Einheit.

Vorzugsweise ist das Schiebeelement 3 mit einer Schnappsicherung versehen, welche im eingeschobenen Zustand des Schiebeelements 3 eingeschnappt ist und ein Verschieben des Schiebelements 3 verhindert. Im hier dargestellten Beispiel ist dies ein Widerhaken 31, welcher in eine Rastnase 20 der Kassette 2 eingreift.

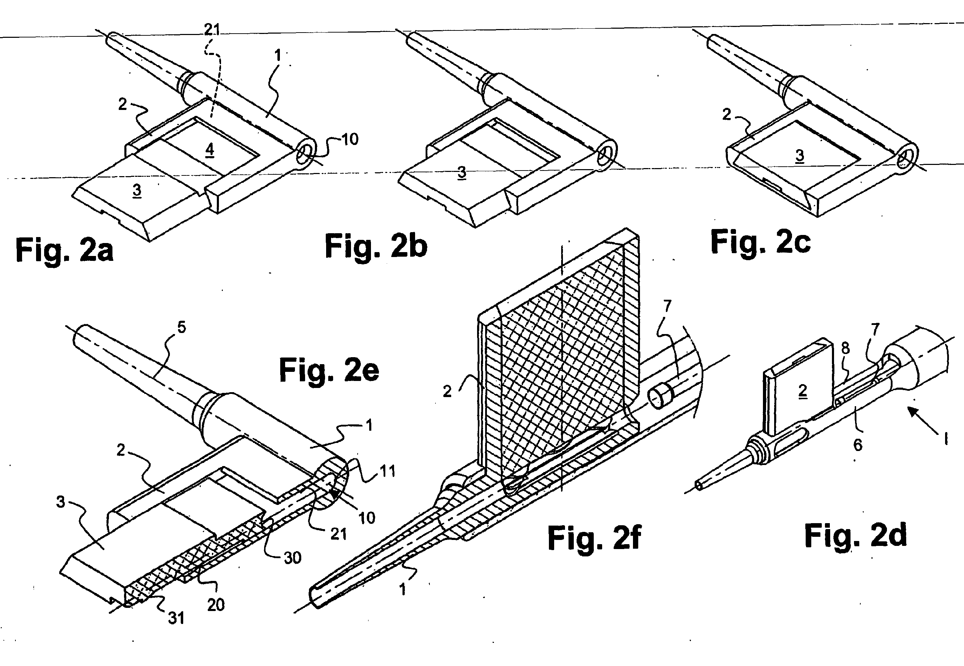

In den Figuren 2a bis 2e ist eine zweite nicht zur Erfindung gehörende Kartusche dargestellt. Die Funktionsweise ist im wesentlichen dieselbe und wird hier nicht wiederholt. Im Gegensatz zur bisher beschriebenen Ausführungsform sind jedoch die Kassette 2 und das Schiebeelement 3 auf derselben Längsseite der Kartusche angeordnet und die Auflagefläche 4 wird durch die Kassette 2 gebildet. Es ist in diesem zweiten Beispiel auch möglich, die Auflagefläche 4 nach wie vor auf dem Schiebeelement 3 anzuordnen, so dass sie gemeinsam mit diesem in die Kassette 2 einschiebbar ist.

Die Kartusche weist eine Führungsfläche 21 auf, unter welcher die Linse L zwecks Rollung oder Faltung geführt entlang gleitet. Diese Führungsfläche 21 kann am Grundkörper 1, bzw. wie hier dargestellt, an der Kassette 2 angeordnet sein. Im hier beschriebenen Beispiel ist die Führungsfläche 21 eine innere, mindestens annähernd plane Oberfläche der Kassette 2.

Zumindest der Grundkörper 1 ist in den oben beschriebenen ersten zwei Ausführungsbeispielen einstückig ausgebildet. In beiden Beispielen ist die Kassette 2 zudem vorzugsweise ebenfalls einstückig am Grundkörper 1 angeformt und bildet somit einen integralen Bestandteil desselben.

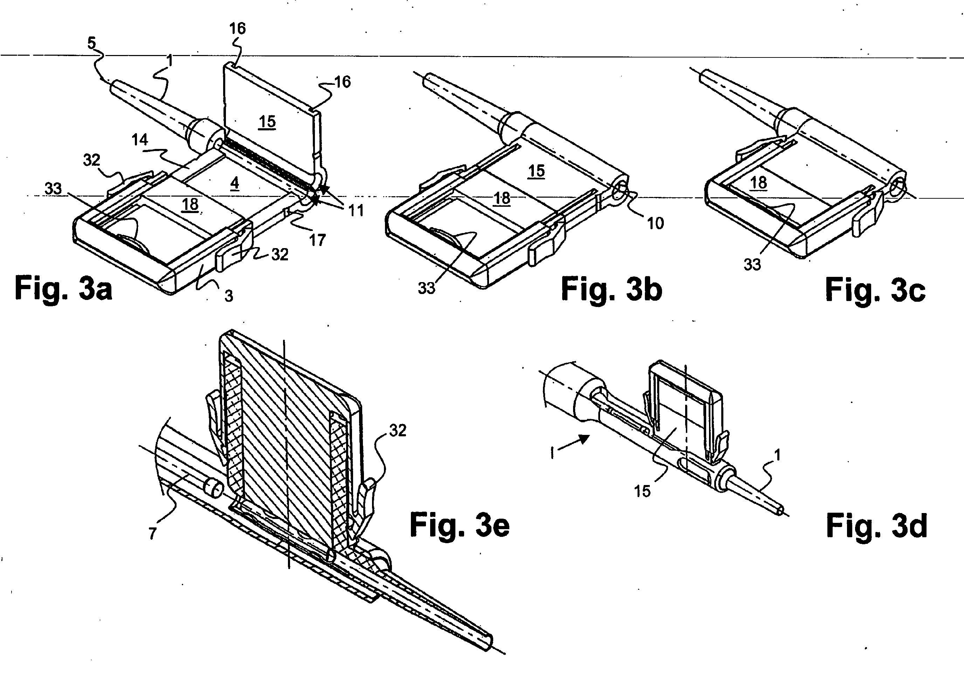

In den Figuren 3a bis 3d ist ein erfindungsgemässes Ausführungsbeispiel dargestellt. Auch hier ist die Funktionsweise wiederum dieselbe. Diese Ausführungsform weist jedoch den Vorteil auf, dass sich die Kartusche bei eingeschobenem Schiebeelement 3 nochmals öffnen lässt und die Lage und Unversehrtheit der gefalteten bzw. gerollten Linse überprüft werden kann.

Diese Kartusche weist einen Grundkörper 1 auf, an welchem zwei parallel zur Längsachse 5 schwenkbare Flügel 14, 15 angeordnet sind. Diese Flügel 14, 15 stehen plattenförmig einer Längsseite des Grundkörpers 1 vor. An einem ersten dieser zwei Flügel 14 ist das Schiebeelement 3 angebracht. Der erste Flügel 14 bildet die Auflagefläche 4. Der zweite Flügel 15 auf den ersten Flügel 14 klappbar, so dass eine aufgelegte ungefaltete Linse L zwischen den zwei Flügeln 14, 15 angeordnet ist. Das Schiebeelement 3 ist zwischen die zwei zusammengeklappten Flügel 14, 15 zwecks Rollung oder Faltung der Linse L einschiebbar.

Mindestens einer der Flügel 14, 15, vorzugsweise beide, weisen äussere Führungsnuten 16 auf, entlang welcher das Schiebeelement 3 geführt verschiebbar ist.

Das Schiebeelement 3 ist an mindestens einer Seite, vorzugsweise an zwei gegenüberliegenden Seiten mit Rastklinken 32 versehen, welche im eingeschobenen Zustand in seitlich an den Flügeln 14, 15 angebrachten Rastnuten 17 eingreifen. Diese Rastklinken 32 sind vorzugsweise lösbar ausgestaltet. Einer der zwei Flügel, vorzugsweise der zweite Flügel 15, lässt sich im eingeschobenen Zustand des Schiebeelements 3 wieder aufklappen, um mindestens teilweise eine Sicht auf die eingerollte oder gefaltete Linse L freizugeben.

Ferner verfügt der erste Flügel 14 über ein Anschlagelement 18, hier einen erhöhten Abschlussbereich, an welches das Schiebeelement 3 im eingeschobenen Zustand ansteht. Im Schiebeelement 3 ist ein Federelement 33 angeordnet, welches einen gefederten Anschlag ermöglicht. In Kombination mit den als Schnappelement wirkenden Rastklinken 32 lässt sich somit gewährleisten, dass die Durchgangsöffnung 10 der Kartusche mit der Durchgangsöffnung 8 des Injektors I massgenau fluchtet.

Mittels dieser Kartuschen lässt sich eine intraokulare, künstliche Linse auf einfache Weise und ohne die Linse zu beschädigen in ein möglichst kleines Format bringen. Des weiteren lässt sich die zusammengefaltete Linse in der Kartusche im Injektor optimal positionieren.

### Bezugszeichenliste

- 1: Grundkörper
- 10: Durchgangsöffnung
- 11: Gewölbte Fläche
- 12: Nut
- 13: Obere Anschlagkante
- 14: Erster Flügel
- 15: Zweiter Flügel
- 16: Äussere Führungsnut
- 17: Rastnut
- 18: Anschlagelement

- 2: Kassette
- 20: Rastnase
- 21: Führungsfläche

- 3: Schiebeelement
- 30: Stirn- oder Führungsfläche
- 31: Widerhaken
- 32: Rastklinke
- 33: Federelement

- 4: Auflagefläche
- 5: Längsachse

- 6: Injektorgehäuse
- 7: Injektorkolben
- 8: Durchgangsöffnung des Injektors

- I: Injektor
- L: Linse

## Patentansprüche

1. Kartusche für eine intraokulare Linse (L) zur Verwendung in einem Injektor (I), wobei die Kartusche aufweist:
einen ein- oder mehrstufigen hohlen, kreiszylinderförmigen Grundkörper (1),
eine mindestens annähernd plane Auflagefläche (4) zur Auflage der Linse (L) in einem teil- oder ungefalteten Zustand,
eine gewölbte Fläche (11), welche an die Auflagefläche (4) angrenzt und
ein Schiebeelement (3), mittels welchem die auf der Auflagefläche (4) aufliegende teil- oder ungefaltete Linse (L) der gewölbten Fläche (11) entlang schiebbar und faltbar, insbesondere rollbar, ist,
**dadurch gekennzeichnet, dass** am Grundkörper (1) zwei parallel zur Längsachse schwenkbare Flügel (14, 15) angeordnet sind, welche plattenförmig einer Längsseite des Grundkörpers (1) vorstehen und wobei an einem ersten dieser zwei Flügel (14, 15) das Schiebeelement (3) angeordnet ist.

2. Kartusche nach Anspruch 1, wobei die Kartusche eine Durchgangsöffnung (10) aufweist, welche bei Verwendung im Injektor (I) mit einer Durchgangsöffnung (8) des Injektors (I) fluchtet und durch welche die Linse (L) in ihrem gefalteten bzw. zusammengerollten Zustand in ein Patientenauge injizierbar ist, und wobei die gewölbte Fläche (11) mindestens einen Teil der Durchgangsöffnung (10) der Kartusche bildet.

3. Kartusche nach Anspruch 1 oder 2, wobei die Kartusche eine Längsachse (5) aufweist, entlang welcher die Linse (L) in ein Patientenauge injizierbar ist und wobei das Schiebeelement (3) in einer Ebene senkrecht zu dieser Längsachse (5) verschiebbar ist.

4. Kartusche nach einem der vorhergehenden Ansprüche, wobei das Schiebeelement (3) eine Führungsfläche (30) zum Schieben der Linse (L) aufweist, wobei die Führungsfläche (30) mindestens eine aus der Gruppe der folgenden Eigenschaften aufweist: sie ist gebogen ausgebildet, sie ist mit einer Beschichtung versehen, sie ist aus Kunststoff gefertigt.

5. Kartusche nach einem der vorhergehenden Ansprüche, wobei das Schiebeelement (3) mit einer Schnappsicherung (20, 31; 17, 32) versehen ist.

6. Kartusche nach einem der vorhergehenden Ansprüche, wobei der erste Flügel (14) die Auflagefläche (4) bildet und der zweite Flügel (15) zum ersten Flügel (14) hin klappbar ist, so dass die auf die Auflagefläche (4) aufgelegte Linse (L) zwischen den zwei Flügeln (14, 15) gehalten ist, und wobei das Schiebeelement (3) zwischen die zwei zusammengeklappten Flügel (14, 15) zwecks Rollung oder Faltung der Linse (L) einschiebbar ist.

7. Kartusche nach Anspruch 6, wobei mindestens einer der Flügel, vorzugsweise beide Flügel (14, 15), äussere Führungsnuten (16) aufweisen, entlang welcher das Schiebeelement (3) geführt verschiebbar ist.

8. Kartusche nach einem der vorhergehenden Ansprüche, wobei das Schiebeelement (3) an mindestens einer Seite, vorzugsweise an zwei gegenüberliegenden Seiten, mit Rastklinken (32) versehen ist, welche im eingeschobenen Zustand in seitlich an den Flügeln (14, 15) angebrachte Rastnuten (17) eingreifen.

9. Kartusche nach Anspruch 8, wobei die Rastklinken (32) lösbar sind.

10. Kartusche nach einem der Ansprüche 6 bis 9, wobei das Schiebeelement (3) im eingeschobenen Zustand gegen ein Anschlagelement (18) des ersten Flügels (14) gefedert ansteht.

## Claims

1. A cartridge for an intraocular lens (L) for use in an injector (I), wherein the cartridge comprises:
a single-step or multiple-step hollow, regular cylindrical base body (1),
an at least approximately plane resting surface (4) for supporting the lens (L) in a partially folded or non-folded state,
an arched surface (11) which adjoins the resting surface (4), and
a sliding element (3) with which the partially folded or non-folded lens (L) supported on the resting surface (4) can be slid along the arched surface (11) and folded, in particular rolled,
**characterized in that** two wings (14, 15) are arranged on the base body (1) so as to swivel parallel to the longitudinal axis, said wings protruding like plates on a longitudinal side of the base body (1), and wherein the sliding element (3) is arranged on a first of these two wings (14, 15).

2. The cartridge as claimed in claim 1, wherein the cartridge has a through-hole (10) which, during use in the injector (I), is flush with a through-hole (8) of the injector (I) and through which the lens (L) in its folded or rolled state can be injected into a patient's eye, and wherein the arched surface (11) forms at least part of the through-hole (10) of the cartridge.

3. The cartridge as claimed in claim 1 or 2, wherein the cartridge has a longitudinal axis (5) along which the lens (L) can be injected into a patient's eye, and wherein the sliding element (3) can be displaced in a plane perpendicular to said longitudinal axis (5).

4. The cartridge as claimed in one of the preceding claims, wherein the sliding element (3) has a guiding surface (30) for sliding the lens (L), wherein the guiding surface (30) has at least one of the properties from the following group: it has a curved design, it is provided with a coating, it is made of plastic.

5. The cartridge as claimed in one of the preceding claims, wherein the sliding element (3) is provided with a snap-fit safety device (20, 31; 17, 32).

6. The cartridge as claimed in one of the preceding claims, wherein the first wing (14) forms the resting surface (4), and the second wing (15) can be folded onto the first wing (14) so that the lens (L) supported on the resting surface (4) is held between the two wings, and wherein the sliding element (3) can be pushed in between the two folded-together wings (14, 15) for the purpose of rolling or folding the lens (L).

7. The cartridge as claimed in claim 6, wherein at least one of the wings, preferably both wings (14, 15), have outer guiding grooves (16) along which the sliding element (3) can be displaced in a guided manner.

8. The cartridge as claimed in one of the preceding claims, wherein the sliding element (3) is provided, on at least one side, preferably on two opposite sides, with snap-fit catches (32) which, in the inserted state, engage in snap-fit grooves (17) arranged laterally on the wings (14, 15).

9. The cartridge as claimed in claim 8, wherein the snap-fit catches (32) are unlockable.

10. The cartridge as claimed in one of claims 6 through 9, wherein the sliding element (3), in the inserted state, rests elastically against a stop element (18) of the first wing (14).

## Revendications

1. Cartouche pour une lentille intraoculaire (L) pour l'utilisation dans un injecteur (I), la cartouche présentant :
un corps de base (1) à un ou plusieurs étages, creux, de forme cylindrique circulaire,
une surface d'appui (4) au moins sensiblement plane, pour l'appui de la lentille (L) dans un état partiellement plié ou non plié,
une surface courbe (11) qui est adjacente à la surface d'appui (4) et
un élément de coulissement (3) au moyen duquel la lentille (L) partiellement pliée ou non pliée s'appliquant sur la surface d'appui (4) peut être coulissée le long de la surface courbe (11) et pliée, notamment roulée,
**caractérisée en ce que** deux ailes (14, 15) pivotant parallèlement par rapport à l'axe longitudinal sont disposées sur le corps de base (1), lesquelles font saillie en forme de plaque sur un côté longitudinal du corps de base (1) et l'élément de coulissement (3) étant disposé sur une première de ces deux ailes (14, 15).

2. Cartouche selon la revendication 1, dans laquelle la cartouche présente une ouverture de passage (10) qui est en affleurement avec une ouverture de passage (8) de l'injecteur (I) lors de l'utilisation dans l'injecteur (I), et à travers laquelle la lentille (L) peut être injectée dans l'oeil d'un patient dans son état plié ou roulé, et dans laquelle la surface courbe (11) forme au moins une partie de l'ouverture de passage (10) de la cartouche.

3. Cartouche selon la revendication 1 ou 2, dans laquelle la cartouche présente un axe longitudinal (5) le long duquel la lentille (L) peut être injectée dans l'oeil d'un patient et dans laquelle l'élément de coulissement (3) peut être coulissé dans un plan perpendiculaire à cet axe longitudinal (5).

4. Cartouche selon l'une quelconque des revendications précédentes, dans laquelle l'élément de coulissement (3) présente une surface de guidage (30) pour faire glisser la lentille (L), la surface de guidage (30) présentant au moins une propriété parmi le groupe des propriétés suivantes : elle a une forme courbe, elle est pourvue d'un revêtement et elle est fabriquée en plastique.

5. Cartouche selon l'une quelconque des revendications précédentes, dans laquelle l'élément de coulissement (3) est pourvu d'une fixation par encliquetage (20, 31 ; 17, 32).

6. Cartouche selon l'une quelconque des revendications précédentes, dans laquelle la première aile (14) forme la surface d'appui (4) et la deuxième aile (15) peut être rabattue par rapport à la première aile (14), de sorte que la lentille (L) posée sur la surface d'appui (4) soit maintenue entre les deux ailes (14, 15), et dans laquelle l'élément de coulissement (3) peut être enfoncé entre les deux ailes rabattues (14, 15) en vue de rouler ou de plier la lentille (L).

7. Cartouche selon la revendication 6, dans laquelle au moins l'une des ailes, de préférence les deux ailes (14, 15), présentent des rainures de guidage extérieures (16) le long desquelles l'élément de coulissement (3) peut coulisser de manière guidée.

8. Cartouche selon l'une quelconque des revendications précédentes, dans laquelle l'élément de coulissement (3) est pourvu sur au moins un côté, de préférence sur deux côtés opposés, de cliquets (32) qui viennent en prise dans l'état enfoncé dans des rainures d'encliquetage (17) prévues latéralement sur les ailes (14, 15).

9. Cartouche selon la revendication 8, dans laquelle les cliquets (32) peuvent être détachés.

10. Cartouche selon l'une quelconque des revendications 6 à 9, dans laquelle l'élément de coulissement (3) s'applique dans l'état enfoncé de manière élastique contre un élément de butée (18) de la première aile (14).
